# EUROPEAN PATENT SPECIFICATION

(11) **EP 2 370 621 B1**
(45) Date of publication and mention of the grant of the patent: **23.09.2015**
(21) Application number: 09834202.5
(22) Date of filing: 05.11.2009
(51) Int. Cl.: D04H 1/42, D04H 13/00, A61F 13/53

(54) **CONDUCTIVE WEBS AND PROCESS FOR MAKING SAME**
LEITFÄHIGE NETZE UND VERFAHREN ZU IHRER HERSTELLUNG
BANDES CONDUCTRICES ET PROCÉDÉ DE FABRICATION ASSOCIÉ

(30) Priority: 22.12.2008 US 341419
(43) Date of publication of application: 05.10.2011
(73) Proprietor: Kimberly-Clark Worldwide, Inc., Neenah, Wisconsin 54956 (US)
(72) Inventor: ALES, Thomas, Michael, Neenah, Wisconsin 54956 (US); NHAN, Davis-Dang, H., Appleton, Wisconsin 54915 (US); SHUKOSKI, Duane, Joseph, Neenah, Wisconsin 54956 (US); REKOSKE, Michael, John, Appleton, WI 54914 (US)
(74) Representative: Davies, Christopher Robert
(86) International application number: PCT/IB2009/054928
(87) International publication number: WO 2010/073133

(56) References cited:
- WO-A2-2009/016528
- JP-A- 2002 266 216
- JP-A- 2002 266 217
- US-A- 4 857 377
- US-A- 5 004 511
- US-A- 5 736 009
- US-A1- 2006 094 320

## Description

### BACKGROUND

Absorbent articles such as diapers, training pants, incontinence products, feminine hygiene products, swim undergarments, and the like conventionally include a liquid permeable body-side liner, a liquid impermeable outer cover, and an absorbent core. The absorbent core is typically located in between the outer cover and the liner for taking in and retaining liquids (e.g., urine) exuded by the wearer.

The absorbent core can be made of, for instance, superabsorbent particles. Many absorbent articles, especially those sold under the tradename HUGGIES™ by the Kimberly-Clark Corporation, are so efficient at absorbing liquids that it is sometimes difficult to tell whether or not the absorbent article has been insulted with a body fluid.

Accordingly, various types of moisture or wetness indicators have been suggested for use in absorbent articles. The wetness indicators may include alarm devices that are designed to assist parents or attendants identify a wet diaper condition early on. The devices can produce an audible signal.

In the past, for instance, wetness indicators have included an open circuit incorporated into the absorbent article that is attached to a power supply and an alarm device. When a conductive substance, such as urine, is detected in the absorbent article, the open circuit becomes closed causing the alarm device to activate. The open circuit may comprise, for instance, two conductive elements that may be made from a metal wire or foil.

Problems have been experienced, however, in efficiently and reliability incorporating wetness indicators into absorbent articles at the process speeds at which absorbent articles are produced. Thus, a need exists for improved wetness sensors that can be easily incorporated into absorbent articles.

In addition, a need also exists for conductive elements for use in a wetness indicator that are made from non-metallic materials. Incorporating metallic components into an absorbent article, for instance, may cause various problems. For instance, once the absorbent articles are packaged, the absorbent articles are typically exposed to a metal detector to ensure that no metallic contaminants have accidentally been included in the package. Making the conductive elements of a wetness indicator from a metal, however, may cause a metal detector to indicate a false positive. The incorporation of metal conductive elements into an absorbent article may also cause problems when the wearer is attempting to pass through a security gate that also includes a metal detector.

WO 2009/016528 A2 discloses a conductive nonwoven web containing pulp fibres combined with conductive fibres.

### SUMMARY

The present invention provides a process for producing a conductive paper web as claimed in claim 1.

The present disclosure is directed to a process for producing a conductive paper web. The process includes the steps of depositing an aqueous suspension of fibers onto a porous forming surface to form a wet web. The aqueous suspension of fibers comprises softwood fibers mixed with carbon fibers. The carbon and softwood fibers can be as described above.

Once deposited onto the porous forming surface, the web can be flattened and then dried. The web can be flattened, for instance, by being fed through calendering rolls. The calendering rolls can apply a pressure of at least about 950 PLI. The web can be dried using any suitable drying device. For instance, in one embodiment, the web can be placed adjacent to one or more drying cylinders that transfer heat to the web. Alternatively, the web can be through-air dried.

After being dried, the web can be slit into a plurality of slits having a width of from about 3 mm to about 10 mm. Each slit can be wound on a separate spool.

Other features and aspects of the present invention are discussed in greater detail below.

### BRIEF DESCRIPTION OF THE DRAWINGS

A full and enabling disclosure of the present invention, including the best mode thereof to one of ordinary skill in the art, is set forth more particularly in the remainder of the specification, including reference to the accompanying figures in which:
Figure 1 is a side view of one embodiment of a process for forming uncreped through-air dried webs in accordance with the present disclosure;
Figure 2 is a side view of another embodiment of a process for forming conductive webs in accordance with the present disclosure;
Figure 3 is a rear perspective view of one embodiment of an absorbent article made in accordance with the present disclosure;
Figure 4 is a front perspective view of the absorbent article illustrated in Figure 3;
Figure 5 is a plan view of the absorbent article shown in Figure 3 with the article in an unfastened, unfolded and laid flat condition showing the surface of the article that faces away from the wearer;
Figure 6 is a plan view similar to Figure 5 showing the surface of the absorbent article that faces the wearer when worn and with portions cut away to show underlying features;
Figure 7 is a perspective view of the embodiment shown in Figure 3 further including one embodiment of a signaling device;
Figure 8 is a side view of still another embodiment of a process for forming conductive webs in accordance with the present disclosure;
Figure 9 is a side view of one embodiment of a process for slitting a nonwoven material made in accordance with the present disclosure into a plurality of slits that are wound on individual spools; and
Figure 10 is a side view of one embodiment of a spool that is used to wind the slits shown in Figure 9.

Repeat use of reference characters in the present specification and drawings is intended to represent same or analogous features or elements of the present disclosure.

### DETAILED DESCRIPTION

It is to be understood by one of ordinary skill in the art that the present discussion is a description of exemplary embodiments only, and is not intended as limiting the broader aspects of the present disclosure.

In general, the present disclosure is generally directed to nonwoven webs containing conductive fibers. The conductive fibers can be incorporated into the web, for instance, such that the web is electrically conductive in at least one direction. For instance, the nonwoven web can be made so that it is capable of carrying an electric current in the length direction, in the width direction, or in any suitable direction.

In accordance with the present disclosure, the conductive nonwoven webs can contain a substantial amount of pulp fibers and can be made using a paper making process. For instance, in one embodiment, the conductive fibers can be combined with pulp fibers and water to form an aqueous suspension of fibers that is then deposited onto a porous surface for forming a conductive tissue web. The conductivity of the tissue web can be controlled by selecting particular conductive fibers, locating the fibers at particular locations within the web and by controlling various other factors and variables. In one embodiment, for instance, the conductive fibers incorporated into the nonwoven web comprise chopped carbon fibers.

After a conductive nonwoven material is made in accordance with the present disclosure, the material can be cut into a plurality of slits that are then wound onto spools. Each slit, for instance, can have a width of from about 1 mm to about 15 mm, such as from about 3 mm to about 10 mm. Once wound onto a spool, each slit can be later incorporated into any suitable product.

Nonwoven webs made in accordance with the present disclosure may be used in numerous different applications. For instance, in one embodiment, the conductive nonwoven material may be incorporated into any suitable electronic device. For instance, the nonwoven web can be used as a fuel cell membrane, as a battery electrode, or may be used in printed electronics. For example, in one particular embodiment, the conductive fibers may form a patterned circuit within the base webs for any suitable end use application.

In one particular embodiment, the conductive nonwoven webs made in accordance with the present disclosure may be used to form wetness sensing devices within absorbent articles. The wetness sensing device, for instance, may be configured to emit a signal, such as an audible signal and/or a visible signal, when a conductive substance, such as urine or fecal matter, is detected in the absorbent article. In one embodiment, for instance, one or more nonwoven webs made in accordance with the present disclosure can be configured to form conductive elements within an absorbent article for creating an open circuit that is configured to close when a conductive substance is present in the article.

The absorbent article may be, for instance, a diaper, a training pant, an incontinence product, a feminine hygiene product, a medical garment, a bandage, and the like. Generally, the absorbent articles containing the open circuit are disposable meaning that they are designed to be discarded after a limited use rather than being laundered or otherwise restored for reuse.

The open circuit contained within the absorbent articles made from nonwoven webs of the present disclosure is configured to be attached to a signaling device. The signaling device can provide power to the open circuit while also including some type of audible and/or visible signal that indicates to the user the presence of a body fluid. Although the absorbent article itself is disposable, the signaling device may be reusable from article to article.

As described above, the base webs of the present disclosure are made by combining conductive fibers with pulp fibers to form nonwoven webs. In one embodiment, a tissue making process or a paper making process is used to form the webs.

The conductive fibers that may be used in accordance with the present disclosure can vary depending upon the particular application and the desired result. Conductive fibers that may be used to form the nonwoven webs include carbon fibers, metallic fibers, conductive polymeric fibers including fibers made from conductive polymers or polymeric fibers containing a conductive material, metal coated fibers, and mixtures thereof. Metallic fibers that may be used include, for instance, copper fibers, aluminum fibers, and the like. Polymeric fibers containing a conductive material include thermoplastic fibers coated with a conductive material or thermoplastic fibers impregnated or blended with a conductive material. For instance, in one embodiment, thermoplastic fibers may be used that are coated with silver.

The conductive fibers incorporated into the nonwoven material can have any suitable length and diameter. In one embodiment, for instance, the conductive fibers can have an aspect ratio of from about 100:1 to about 1,000:1.

The amount of conductive fibers contained in the nonwoven web can vary based on many different factors, such as the type of conductive fiber incorporated into the web and the ultimate end use of the web. The conductive fibers may be incorporated into the nonwoven web, for instance, in an amount from about 1% by weight to about 90% by weight, or even greater. In one embodiment, the conductive fibers can be present in the nonwoven web in an amount from about 5% by weight to about 15% by weight, such as from about 8% by weight to about 12% by weight.

Carbon fibers that may be used in the present disclosure include fibers made entirely from carbon or fibers containing carbon in amounts sufficient so that the fibers are electrically conductive. In one embodiment, for instance, carbon fibers may be used that are formed from a polyacrylonitrile (or PAN) polymer. In particular, the carbon fibers are formed by heating, oxidizing, and carbonizing polyacrylonitrile PAN polymer fibers. Such fibers typically have high purity and contain relatively high molecular weight molecules. For instance, the fibers can contain carbon in an amount greater than about 85% by weight. In one embodiment, for instance, the purity of the carbon fibers can be from about 85% to about 95%, such as from about 88% to about 92%. Although higher purity fibers have better conductive properties, the higher purity fibers can be more expensive. Sufficient electrical characteristics, on the other hand, can be obtained using fibers with the purity ranges described above.

In order to form carbon fibers from polyacrylonitrile PAN polymer fibers, the polyacrylonitrile PAN fibers are first heated in an oxygen environment, such as air. While heating, cyano sites within the polyacrylonitrile PAN polymer form repeat cyclic units of tetrahydropyridine. As heating continues, the polymer begins to oxidate. During oxidation, hydrogen is released causing carbon to form aromatic rings.

After oxidation, the fibers are then further heated in an oxygen starved environment. For instance, the fibers can be heated to a temperature of greater than about 1300°C, such as greater than 1400°C, such as from about 1300°C to about 1800°C. During heating, the fibers undergo carbonization. During carbonization, adjacent polymer chains join together to form a lamellar, basal plane structure of nearly pure carbon.

Polyacrylonitrile-based carbon fibers are available from numerous commercial sources. For instance, such carbon fibers can be obtained from Toho Tenax America, Inc. of Rockwood, Tennessee.

Other raw materials used to make carbon fibers are Rayon and petroleum pitch.

Of particular advantage, the formed carbon fibers can be chopped to any suitable length. In one embodiment of the present disclosure, for instance, chopped carbon fibers may be incorporated into the base web having a length of from about 1 mm to about 6 mm, such as from about 2 mm to about 5 mm. The fibers can have an average diameter of from about 3 microns to about 15 microns, such as from about 5 microns to about 10 microns. In one embodiment, for instance, the carbon fibers may have a length of about 3 mm and an average diameter of about 7 microns.

In one embodiment, the carbon fibers incorporated into the nonwoven base webs have a water soluble sizing. Sizing can be in the amount of 0.1 - 10% by weight. Water soluble sizings, can be, but not limited to, polyamide compounds, epoxy resin ester and poly(vinyl pyrrolidone). In this manner, the sizing is dissolved when mixing the carbon fibers in water to provide a good dispersion of carbon fibers in water prior to forming the nonwoven web. The sizing also assists in handling the fibers, by controlling them from becoming airborne while being added during the process.

In forming conductive nonwoven webs in accordance with the present disclosure, the above conductive fibers are combined with other fibers suitable for use in tissue or paper making processes. The fibers combined with the conductive fibers may comprise any natural or synthetic cellulosic fibers including, but not limited to nonwoody fibers, such as cotton, abaca, kenaf, sabai grass, flax, esparto grass, straw, jute hemp, bagasse, milkweed floss fibers, algae fibers, and pineapple leaf fibers; and woody or pulp fibers such as those obtained from deciduous and coniferous trees, including softwood fibers, such as northern and southern softwood kraft fibers; hardwood fibers, such as eucalyptus, maple, birch, and aspen. Pulp fibers can be prepared in high-yield or low-yield forms and can be pulped in any known method, including kraft, sulfite, high-yield pulping methods and other known pulping methods. Fibers prepared from organosolv pulping methods can also be used, including the fibers and methods disclosed in U.S. Patent No. 4,793,898, issued Dec. 27, 1988 to Laamanen et al.; U.S. Patent No. 4,594,130, issued June 10, 1986 to Chang et al.; and U.S. Patent No. 3,585,104. Useful fibers can also be produced by anthraquinone pulping, exemplified by U.S. Patent No. 5,595,628 issued Jan. 21, 1997, to Gordon et al.

In one embodiment, softwood fibers are used to produce the nonwoven material. Softwood fibers tend to be longer which reduces particulate emission during manufacturing and converting. The longer pulp fibers also have a tendency to entangle better with the conductive fibers, such as the carbon fibers.

The pulp fibers incorporated into the nonwoven material, such as softwood fibers, can also be refined so as to increase the amount of bonding sites on each fiber. The increase in bonding sites increases the mechanical entanglement of the pulp fibers with the conductive fibers in the finished material. This allows for a very flat uniform paper with reduced carbon fiber fallout during processing. The refining action also increases the overall strength of the nonwoven material. For example, in one embodiment, the pulp fibers can have a Canadian Standard Freeness of greater than about 350 mL, such as greater than about 375 mL. For instance, the pulp fibers can be refined so as to have a Canadian Standard Freeness of from about 350 mL to about 600 mL.

A portion of the fibers, such as up to 50% or less by dry weight, or from about 5% to about 30% by dry weight, can be synthetic fibers such as rayon, polyolefin fibers, polyester fibers, polyvinyl alcohol fibers, bicomponent sheath-core fibers, multi-component binder fibers, and the like. An exemplary polyethylene fiber is Pulpex®, available from Hercules, Inc. (Wilmington, DE). Synthetic cellulose fiber types include rayon in all its varieties and other fibers derived from viscose or chemically-modified cellulose.

Incorporating thermoplastic fibers into the nonwoven web may provide various advantages and benefits. For example, incorporating thermoplastic fibers into the web may allow the webs to be thermally bonded to adjacent structures. For instance, the webs may be thermally bonded to other nonwoven materials, such as a diaper liner which may comprise, for instance, a spunbond web or a meltblown web.

Chemically treated natural cellulosic fibers can also be used such as mercerized pulps, chemically stiffened or crosslinked fibers, or sulfonated fibers. For good mechanical properties in using papermaking fibers, it can be desirable that the fibers be relatively undamaged and largely unrefined or only lightly refined. Mercerized fibers, regenerated cellulosic fibers, cellulose produced by microbes, rayon, and other cellulosic material or cellulosic derivatives can be used. Suitable fibers can also include recycled fibers, virgin fibers, or mixtures thereof.

Other papermaking fibers that can be used in the present disclosure include paper broke or recycled fibers and high yield fibers. High yield pulp fibers are those papermaking fibers produced by pulping processes providing a yield of about 65% or greater, more specifically about 75% or greater, and still more specifically about 75% to about 95%. Yield is the resulting amount of processed fibers expressed as a percentage of the initial wood mass. Such pulping processes include bleached chemithermomechanical pulp (BCTMP), chemithermomechanical pulp (CTMP), pressure/pressure thermomechanical pulp (PTMP), thermomechanical pulp (TMP), thermomechanical chemical pulp (TMCP), high yield sulfite pulps, and high yield Kraft pulps, all of which leave the resulting fibers with high levels of lignin. High yield fibers are well known for their stiffness in both dry and wet states relative to typical chemically pulped fibers.

In general, any process capable of forming a tissue or paper web can be utilized in forming the conductive web. For example, a papermaking process of the present disclosure can utilize embossing, wet pressing, air pressing, through-air drying, uncreped through-air drying, hydroentangling, air laying, as well as other steps known in the art. The tissue web may be formed from a fiber furnish containing pulp fibers in an amount of at least 50% by weight, such as at least 60% by weight, such as at least 70% by weight, such as at least 85% by weight.

The nonwoven webs can also be pattern densified or imprinted, such as the tissue sheets disclosed in any of the following U.S. Patent Nos.: 4,514,345 issued on April 30, 1985, to Johnson et al.; 4,528,239 issued on July 9, 1985, to Trokhan; 5,098,522 issued on March 24, 1992; 5,260,171 issued on November 9, 1993, to Smurkoski et al.; 5,275,700 issued on January 4, 1994, to Trokhan; 5,328,565 issued on July 12,1994, to Rasch et al.; 5,334,289 issued on August 2, 1994, to Trokhan et al.; 5,431,786 issued on July 11, 1995, to Rasch et al.; 5,496,624 issued on March 5, 1996, to Steltjes, Jr. et al.; 5,500,277 issued on March 19, 1996, to Trokhan et al.; 5,514,523 issued on May 7, 1996, to Trokhan et al.; 5,554,467 issued on September 10, 1996, to Trokhan et al.; 5,566,724 issued on October 22, 1996, to Trokhan et al.; 5,624,790 issued on April 29, 1997, to Trokhan et al.; and, 5,628,876 issued on May 13,1997, to Ayers et al.. Such imprinted tissue sheets may have a network of densified regions that have been imprinted against a drum dryer by an imprinting fabric, and regions that are relatively less densified (e.g., "domes" in the tissue sheet) corresponding to deflection conduits in the imprinting fabric, wherein the tissue sheet superposed over the deflection conduits was deflected by an air pressure differential across the deflection conduit to form a lower-density pillow-like region or dome in the tissue sheet.

Wet and dry strength agents may be applied or incorporated into the base sheet. As used herein, "wet strength agents" refer to materials used to immobilize the bonds between fibers in the wet state. Typically, the means by which fibers are held together in paper and tissue products involve hydrogen bonds and sometimes combinations of hydrogen bonds and covalent and/or ionic bonds. In the present invention, it may be useful to provide a material that will allow bonding of fibers in such a way as to immobilize the fiber-to-fiber bond points and make them resistant to disruption in the wet state. In the present application, wet strength agents also assist in bonding the conductive fibers, such as the carbon fibers, to the rest of the fibers contained in the web. In this manner, the conductive fibers are inhibited from falling out of the web during further handling.

Any material that when added to a tissue sheet or sheet results in providing the tissue sheet with a mean wet geometric tensile strength/dry geometric tensile strength ratio in excess of about 0.1 will, for purposes of the present invention, be termed a wet strength agent. Typically these materials are termed either as permanent wet strength agents or as "temporary" wet strength agents. For the purposes of differentiating permanent wet strength agents from temporary wet strength agents, the permanent wet strength agents will be defined as those resins which, when incorporated into paper or tissue products, will provide a paper or tissue product that retains more than 50% of its original wet strength after exposure to water for a period of at least five minutes. Temporary wet strength agents are those which show 50% or less than, of their original wet strength after being saturated with water for five minutes. Both classes of wet strength agents find application in the present invention. The amount of wet strength agent added to the pulp fibers may be at least about 0.1 dry weight percent, more specifically about 0.2 dry weight percent or greater, and still more specifically from about 0.1 to about 3 dry weight percent, based on the dry weight of the fibers.

Permanent wet strength agents will typically provide a more or less long-term wet resilience to the structure of a tissue sheet. In contrast, the temporary wet strength agents will typically provide tissue sheet structures that had low density and high resilience, but would not provide a structure that had long-term resistance to exposure to water or body fluids.

The temporary wet strength agents may be cationic, nonionic or anionic. Such compounds include PAREZ™ 631 NC and PAREZ® 725 temporary wet strength resins that are cationic glyoxylated polyacrylamide available from Cytec Industries (West Paterson, New Jersey). This and similar resins are described in U.S. Patent No. 3,556,932, issued on January 19, 1971, to Coscia et al. and U.S. Patent No. 3,556,933, issued on January 19, 1971, to Williams et al. Hercobond 1366, manufactured by Hercules, Inc., located at Wilmington, Delaware, is another commercially available cationic glyoxylated polyacrylamide that may be used in accordance with the present invention. Additional examples of temporary wet strength agents include dialdehyde starches such as Cobond® 1000 from National Starch and Chemical Company and other aldehyde containing polymers such as those described in U.S. Patent No. 6,224,714, issued on May 1, 2001, to Schroeder et al.; U.S. Patent No. 6,274,667, issued on August 14, 2001, to Shannon et al.; U.S. Patent No. 6,287,418, issued on September 11, 2001, to Schroeder et al.; and, U.S. Patent No. 6,365,667, issued on April 2, 2002, to Shannon et al..

Permanent wet strength agents comprising cationic oligomeric or polymeric resins can be used in the present invention. Polyamide-polyamine-epichlorohydrin type resins also referred to as polyaminoamide-epichlorohydrin resins such as KYMENE 557H sold by Hercules, Inc., located at Wilmington, Delaware, are the most widely used permanent wet-strength agents and are suitable for use in the present invention. Such materials have been described in the following U.S. Patent Nos.: 3,700,623, issued on October 24, 1972, to Keim; 3,772,076, issued on November 13, 1973, to Keim; 3,855,158, issued on December 17, 1974, to Petrovich et al.; 3,899,388, issued on August 12, 1975, to Petrovich et al.; 4,129,528, issued on December 12, 1978, to Petrovich et al.; 4,147,586, issued on April 3, 1979, to Petrovich et al.; and, 4,222,921, issued on September 16, 1980, to van Eenam. Other cationic resins include polyethylenimine resins and aminoplast resins obtained by reaction of formaldehyde with melamine or urea. It can be advantageous to use both permanent and temporary wet strength resins in the manufacture of tissue products.

In one embodiment, a relatively large amount of a wet strength agent is incorporated into the nonwoven material. The wet strength agent may also add to the dry strength of the product. In addition, wet strength agents aid in the chemical entangling of the fibers in the material to improve the retention of the conductive fibers. The amount of wet strength agent added to the nonwoven material can depend upon various different factors. In general, for instance, the wet strength agent can be added in an amount from about 1 kg/mton to about 12 kg/mton, such as from about 5 kg/mton to about 10 kg/mton. In certain embodiments, it may be desirable to add as much wet strength agent as possible. In these embodiments, for instance, the wet strength agent can be added in amounts greater than about 7 kg/mton, such as in amounts greater than about 8 kg/mton.

Dry strength agents are well known in the art and include but are not limited to modified starches and other polysaccharides such as cationic, amphoteric, and anionic starches and guar and locust bean gums, modified polyacrylamides, carboxymethylcellulose, sugars, polyvinyl alcohol, chitosans, and the like. Such dry strength agents are typically added to a fiber slurry prior to tissue sheet formation or as part of the creping package.

Additional types of chemicals that may be added to the nonwoven web include, but is not limited to, absorbency aids usually in the form of cationic, anionic, or non-ionic surfactants, humectants and plasticizers such as low molecular weight polyethylene glycols and polyhydroxy compounds such as glycerin and propylene glycol. Materials that supply skin health benefits such as mineral oil, aloe extract, vitamin E, silicone, lotions in general and the like may also be incorporated into the finished products.

In general, the products of the present disclosure can be used in conjunction with any known materials and chemicals that are not antagonistic to its intended use. Examples of such materials include but are not limited to baby powder, baking soda, chelating agents, zeolites, perfumes or other odor-masking agents, cyclodextrin compounds, oxidizers, and the like. Of particular advantage, when carbon fibers are used as the conductive fibers, the carbon fibers also serve as odor absorbents. Superabsorbent particles, synthetic fibers, or films may also be employed. Additional options include dyes, optical brighteners, humectants, emollients, and the like.

Nonwoven webs made in accordance with the present disclosure can include a single homogeneous layer of fibers or may include a stratified or layered construction. For instance, the nonwoven web ply may include two or three layers of fibers. Each layer may have a different fiber composition. The particular fibers contained in each layer generally depends upon the product being formed and the desired results. In one embodiment, for instance, a middle layer contains pulp fibers in combination with the conductive fibers. The outer layers, on the other hand, can contain only pulp fibers, such as softwood fibers and/or hardwood fibers.

In one embodiment, nonwoven webs made in accordance with the present disclosure are generally made according to a wetlaid process. In this embodiment, the fibers are combined with water to form an aqueous suspension and then deposited onto a porous forming surface where a wet web is formed. In one embodiment, an aqueous suspension containing the pulp fibers is first produced. The conductive fibers, such as the carbon fibers, are then injected into the aqueous suspension of pulp fibers prior to depositing the aqueous suspension onto the forming surface. For example, the conductive fibers can be injected into the aqueous suspension of pulp fibers in a headbox just prior to depositing the fibers onto the forming surface. The aqueous suspension of pulp fibers, for instance, may contain greater than 99% by weight water. For instance, in one embodiment, the aqueous suspension of pulp fibers contains the pulp fibers in an amount of less than 1% by weight, such as in an amount of about 0.5% by weight. The conductive fibers can then be injected into the aqueous suspension at a similar dilution. For instance, an aqueous suspension of carbon fibers containing carbon fibers in an amount of about 0.5% by weight may be injected into the aqueous suspension of pulp fibers.

Injecting the conductive fibers into an aqueous suspension of pulp fibers has been found to reduce the formation of flocks of the carbon fibers. It has been discovered that flocks have a greater tendency to form when the amount of time the fibers are mixed together increases. The creation of flocks, for instance, can produce weak spots in the resulting material and cause wet breaks when the nonwoven material is later processed.

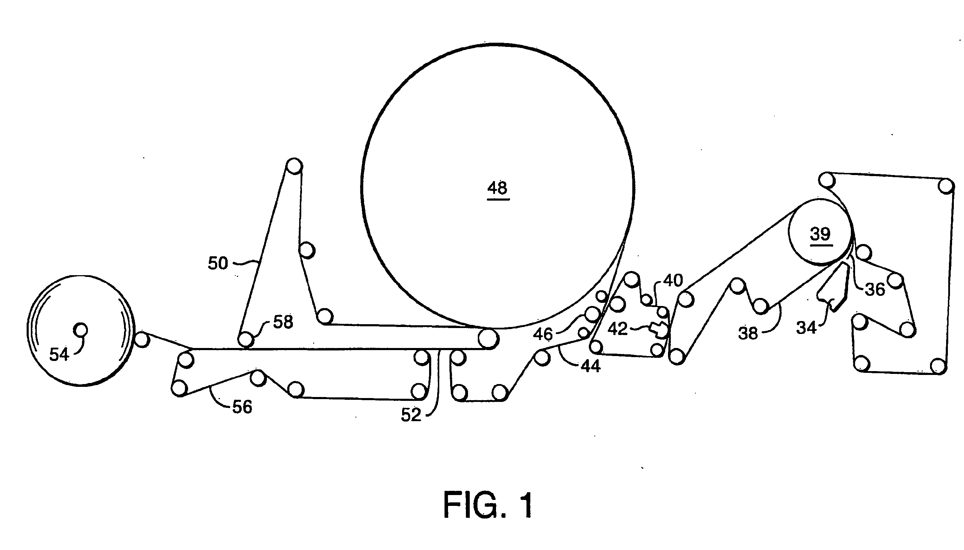

Once the aqueous suspension of fibers is formed into a nonwoven web, the web may be processed using various techniques and methods. For example, referring to Fig. 1, shown is a method for making uncreped, throughdried tissue sheets. In one embodiment, it may be desirable to form the nonwoven web using an uncreped, through-air drying process. It was found that creping the nonwoven web during formation may cause damage to the conductive fibers by destroying the network of conductive fibers within the nonwoven web. Thus, the nonwoven web becomes non-conductive.

For simplicity, the various tensioning rolls schematically used to define the several fabric runs are shown, but not numbered. It will be appreciated that variations from the apparatus and method illustrated in **Fig. 1** can be made without departing from the general process. Shown is a twin wire former having a papermaking headbox **34,** such as a layered headbox, which injects or deposits a stream **36** of an aqueous suspension of papermaking fibers onto the forming fabric **38** positioned on a forming roll **39.** The forming fabric serves to support and carry the newly-formed wet web downstream in the process as the web is partially dewatered to a consistency of about 10 dry weight percent. Additional dewatering of the wet web can be carried out, such as by vacuum suction, while the wet web is supported by the forming fabric.

The wet web is then transferred from the forming fabric to a transfer fabric **40.** In one optional embodiment, the transfer fabric can be traveling at a slower speed than the forming fabric in order to impart increased stretch into the web. This is commonly referred to as a "rush" transfer. The relative speed difference between the two fabrics can be from 0-15 percent, more specifically from about 0-8 percent. Transfer is preferably carried out with the assistance of a vacuum shoe **42** such that the forming fabric and the transfer fabric simultaneously converge and diverge at the leading edge of the vacuum slot.

The web is then transferred from the transfer fabric to the throughdrying fabric **44** with the aid of a vacuum transfer roll **46** or a vacuum transfer shoe, optionally again using a fixed gap transfer as previously described. The throughdrying fabric can be traveling at about the same speed or a different speed relative to the transfer fabric. If desired, the throughdrying fabric can be run at a slower speed to further enhance stretch. Transfer can be carried out with vacuum assistance to ensure deformation of the sheet to conform to the throughdrying fabric, thus yielding desired bulk and appearance if desired. Suitable throughdrying fabrics are described in U.S. Patent No. 5,429,686 issued to Kai F. Chiu et al. and U. S. Patent No. 5,672,248 to Wendt, et al..

In one embodiment, the throughdrying fabric provides a relatively smooth surface. Alternatively, the fabric can contain high and long impression knuckles.

The side of the web contacting the throughdrying fabric is typically referred to as the "fabric side" of the nonwoven web. The fabric side of the web, as described above, may have a shape that conforms to the surface of the throughdrying fabric after the fabric is dried in the throughdryer. The opposite side of the paper web, on the other hand, is typically referred to as the "air side". The air side of the web is typically smoother than the fabric side during normal throughdrying processes.

The level of vacuum used for the web transfers can be from about 3 to about 15 inches of mercury (75 to about 380 millimeters of mercury), preferably about 5 inches (125 millimeters) of mercury. The vacuum shoe (negative pressure) can be supplemented or replaced by the use of positive pressure from the opposite side of the web to blow the web onto the next fabric in addition to or as a replacement for sucking it onto the next fabric with vacuum. Also, a vacuum roll or rolls can be used to replace the vacuum shoe(s).

While supported by the throughdrying fabric, the web is finally dried to a consistency of about 94% or greater by the throughdryer **48** and thereafter transferred to a carrier fabric **50.** The dried basesheet **52** is transported to the reel **54** using carrier fabric **50** and an optional carrier fabric **56.** An optional pressurized turning roll **58** can be used to facilitate transfer of the web from carrier fabric **50** to fabric **56.** Suitable carrier fabrics for this purpose are Albany International 84M or 94M and Asten 959 or 937, all of which are relatively smooth fabrics having a fine pattern. Although not shown, reel calendering or subsequent off-line calendering can be used to improve the smoothness and softness of the basesheet. Calendering the web may also cause the conductive fibers to orient in a certain plane or in a certain direction. For instance, in one embodiment, the web can be calendered in order to cause primarily all of the conductive fibers to lie in the X-Y plane and not in the Z direction. In this manner, the conductivity of the web can be improved while also improving the softness of the web.

In one embodiment, the nonwoven web **52** is a web which has been dried in a flat state. For instance, the web can be formed while the web is on a smooth throughdrying fabric. Processes for producing uncreped throughdried fabrics are, for instance, disclosed in U. S. Patent No. 5,672,248 to Wendt, et al.; U. S. Patent No. 5,656,132 to Farrington, et al.; U. S. Patent No. 6,120,642 to Lindsay and Burazin; U. S. Patent No. 6,096,169 to Hermans, et al.; U. S. Patent No. 6,197,154 to Chen, et al.; and U. S. Patent No. 6,143,135 to Hada, et al..

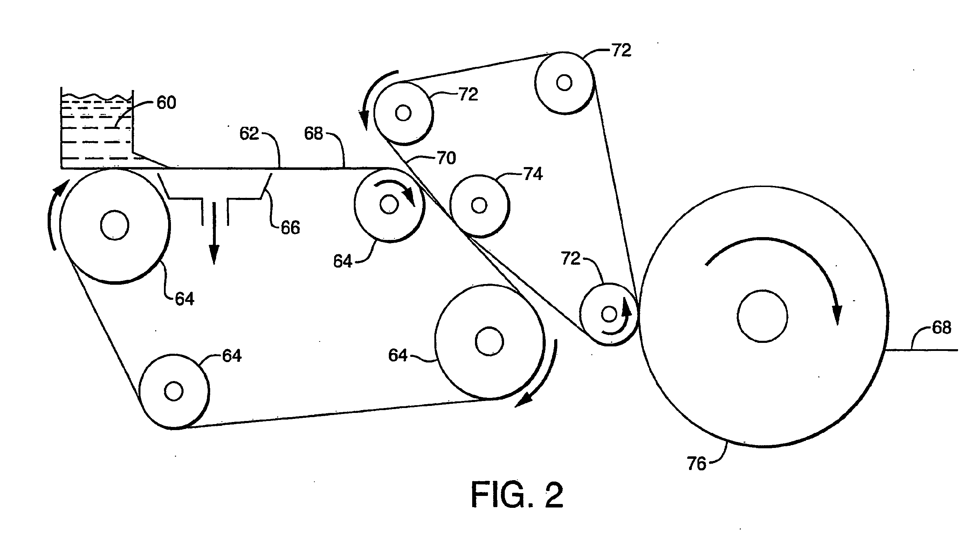

In **Fig. 1****,** a process is shown for producing uncreped through-air dried webs. It should be understood, however, that any suitable process or technique that does not use creping may be used to form the conductive nonwoven web. For example, referring to **Fig. 2****,** another process that may be used to form nonwoven webs in accordance with the present disclosure is shown. In the embodiment illustrated in **Fig. 2****,** the newly formed web is wet pressed during the process.

In this embodiment, a headbox **60** emits an aqueous suspension of fibers onto a forming fabric **62** which is supported and driven by a plurality of guide rolls **64.** The headbox **60** may be similar to the headbox **34** shown in **Fig. 1****.** In addition, the aqueous suspension of fibers may contain conductive fibers as described above. A vacuum box **66** is disposed beneath forming fabric **62** and is adapted to remove water from the fiber furnish to assist in forming a web. From forming fabric **62,** a formed web **68 is** transferred to a second fabric **70,** which may be either a wire or a felt. Fabric **70** is supported for movement around a continuous path by a plurality of guide rolls **72.** Also included is a pick up roll **74** designed to facilitate transfer of web **68** from fabric **62** to fabric **70.**

From fabric **70,** web **68,** in this embodiment, is transferred to the surface of a rotatable heated dryer drum **76,** such as a Yankee dryer. As shown, as web **68** is carried through a portion of the rotational path of the dryer surface, heat is imparted to the web causing most of the moisture contained within the web to be evaporated. The web **68** is then removed from the dryer drum **76** without creping the web.

In order to remove the web **68** from the dryer drum **76,** in one embodiment, a release agent may be applied to the surface of the dryer drum or to the side of the web that contacts the dryer drum. In general, any suitable release agent may be used that facilitates removal of the web from the drum so as to avoid the necessity of creping the web.

Release agents that may be used include, for instance, polyamidoamine epichlorohydrin polymers, such as those sold under the trade name REZOSOL by the Hercules Chemical Company. Particular release agents that may be used in the present disclosure include Release Agent 247, Rezosol 1095, Crepetrol 874, Rezosol 974, ProSoft TQ-1003 all available from the Hercules Chemical Company, Busperse 2032, Busperse 2098, Busperse 2091, Buckman 699 all available from Buckman Laboratories, and 640C release, 640D release, 64575 release, DVP4V005 release, DVP4V008 release all available from Nalco.

During the process of making the nonwoven material, such as either shown in **FIG. 1** or **FIG. 2****,** the web can be flattened and densified. One technique for flattening or densifying the web is by feeding the web through the nip of opposing calender rolls. Flattening and densifying the sheet has been found to reduce fallout of the carbon fiber during further processing. Flattening the web reduces the overall caliper or thickness and increases the electrical conductivity of the material by increasing the conductive fiber network and uniformity. Reducing the thickness of the material may also increase the run time of material rolls during product processing which improves efficiency, waste and delay. Increased conductivity may allow for an overall reduction in conductive fiber contained in the finished material.

When calendering the web, the web can be calendered in a dry state or in a wet state. In one embodiment, for instance, the calender rolls may apply a pressure of at least 900 PLI, such as from about 900 PLI to about 1100 PLI. For instance, in one particular embodiment, the pressure applied by the calendering rolls may be from about 950 PLI to about 1000 PLI, such as a pressure of about 980 PLI.

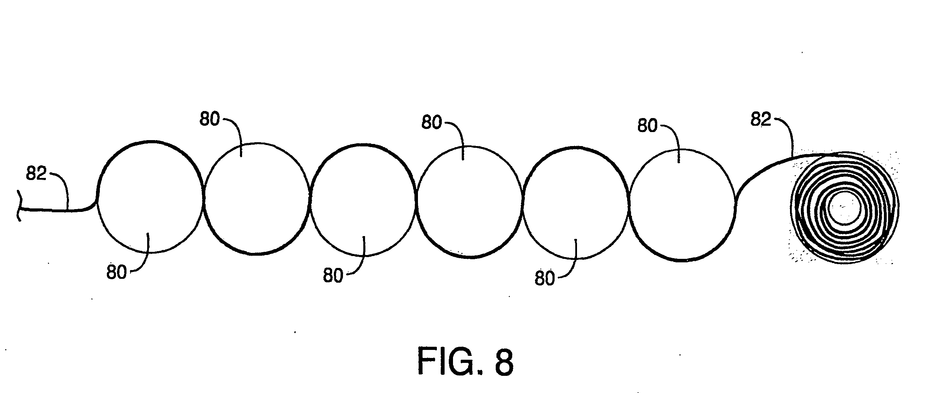

In an alternative embodiment, as shown in **Fig. 8****,** the web can be pressed against a plurality of drying cylinders that not only dry the web but flatten and densify the web. For example, referring to **Fig. 8****,** a plurality of consecutive drying cylinders **80** are shown. In this embodiment, six consecutive drying cylinders are illustrated. It should be understood, however, that in other embodiments more or less drying cylinders may be used. For example, in one embodiment, eight to twelve consecutive drying cylinders may be incorporated into the process.

As shown, a wet web **82** formed according to any suitable process is pressed into engagement with the first drying cylinder **80.** For example, in one embodiment, a fabric or suitable conveyor may be used to press the web against the surface of the drying cylinder. The web is wrapped around the drying cylinder at least about 150°, such as at least about 180° prior to being pressed into engagement with the second drying cylinder. Each of the drying cylinders can be heated to an optimized temperature for drying the web during the process.

Nonwoven webs made in accordance with the present disclosure can have various different properties and characteristics depending upon the application in which the webs are to be used and the desired results. For instance, the nonwoven web can have a basis weight of from about 15 gsm to about 60 gsm or greater. In one embodiment, for instance, the new nonwoven web can have a basis weight of from about 30 gsm to about 40 gsm.

Once densified or flattened, the nonwoven web can be made with a relatively low bulk. For instance, as described above, in some processes, the web can be densified as it is formed. The bulk of these webs, for instance, may be less than about 2 cc/g, such as less than about 1 cc/g, such as less than about 0.5 cc/g.

The sheet "bulk" is calculated as the quotient of the caliper of a dry tissue sheet, expressed in microns, divided by the dry basis weight, expressed in grams per square meter. The resulting sheet bulk is expressed in cubic centimeters per gram. More specifically, the caliper is measured as the total thickness of a stack of ten representative sheets and dividing the total thickness of the stack by ten, where each sheet within the stack is placed with the same side up. Caliper is measured in accordance with TAPPI test method T411 om-89 "Thickness (caliper) of Paper, Paperboard, and Combined Board" with Note 3 for stacked sheets. The micrometer used for carrying out T411 om-89 is an Emveco 200-A Tissue Caliper Tester available from Emveco, Inc., Newberg, Oregon. The micrometer has a load of 2.00 kilo-Pascals (132 grams per square inch), a pressure foot area of 2500 square millimeters, a pressure foot diameter of 56.42 millimeters, a dwell time of 3 seconds and a lowering rate of 0.8 millimeters per second.

Nonwoven webs made in accordance with the present disclosure can also have sufficient strength so as to facilitate handling. For instance, in one embodiment, the webs can have a strength (or peak load) of greater than about 5000 grams force in the machine or length direction, such as greater than about 5500 grams force, such as even greater than about 6000 grams force. Tensile testing of the nonwoven material, for instance, can be conducted on a one inch wide specimen at 300 mm/min and 75 mm gage length.

The conductivity of the nonwoven web can also vary depending upon the type of conductive fibers incorporated into the web, the amount of conductive fibers incorporated into the web, and the manner in which the conductive fibers are positioned, concentrated or oriented in the web. In one embodiment, for instance, the nonwoven web can have a resistance of less than about 1500 Ohms/square, such as less than about 100 Ohms/square, such as less than about 80 Ohms/square. In one embodiment, for instance, the nonwoven material can have a resistance of from about 20 Ohms/square to about 80 Ohms/square, such as from about 20 Ohms/square to about 40 Ohms/square.

The conductivity of the sheet is calculated as the quotient of the resistant measurement of a sheet, expressed in Ohms, divided by the ratio of the length to the width of the sheet. The resulting resistance of the sheet is expressed in Ohms per square. More specifically, the resistance measurement is in accordance with ASTM F1896-98 "Test Method for Determining the Electrical Resistivity of a Printed Conductive Material". The resistance measuring device (or Ohm meter) used for carrying out ASTM F1896-98 is a Fluke multimeter (model 189) equipped with Fluke alligator clips (model AC120); both are available from Fluke Corporation, Everett, Washington.

When using carbon fibers, the resulting nonwoven material is generally gray or black in color. If desired, the material may be dyed a particular shade of color to improve aesthetics. For instance, in one embodiment, the material can be dyed a shade of purple or a shade of blue. Particular dyes that may be used include PANTONE 264U purple dye or PANTONE 291U blue dye.

The resulting conductive web made in accordance with the present disclosure may be used alone as a single ply product or can be combined with other webs or films to form a multi-ply product. In one embodiment, the conductive nonwoven web may be combined with other nonwoven webs to form a 2-ply product or a 3-ply product. The other nonwoven webs, for instance, may be made entirely from pulp fibers and can be made according to any of the processes described above.

In an alternative embodiment, the conductive nonwoven web made according to the present disclosure may be laminated using an adhesive or otherwise to other nonwoven or polymeric film materials. For instance, in one embodiment, the conductive nonwoven web may be laminated to a meltblown web and/or a spunbond web that are made from polymeric fibers, such as polypropylene fibers. As described above, in one embodiment, the conductive nonwoven web can contain synthetic fibers. In this embodiment, the nonwoven web may be bonded to an opposing web containing synthetic fibers such as a meltblown web or spunbond web.

After the conductive nonwoven material of the present disclosure is formed, the material can be wound into a parent roll. The width of the formed material can vary depending upon the tissue or paper making process used. For instance, in general, the material can have a width of from about 60 inches to about 100 inches. In one embodiment, the nonwoven material is then cut into a plurality of slits for later use in various applications. For example, in one embodiment, the material can be slit to a width of from about 3 mm to about 12 mm, such as from about 5 mm to about 8 mm. In particular, the nonwoven material can be slit to a width to maintain strength and electrical properties while minimizing raw material costs.

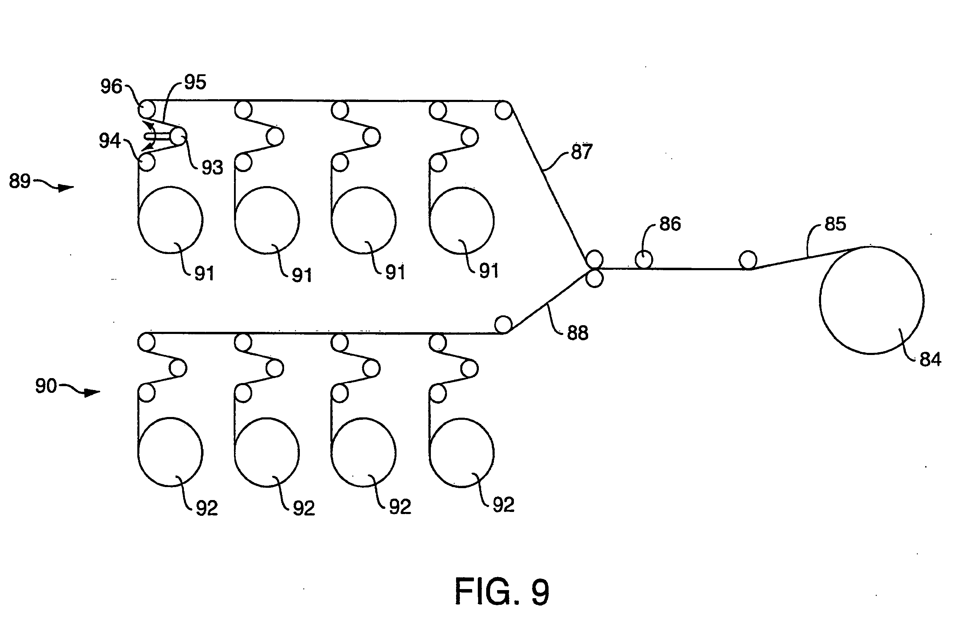

Since slitting of the material can produce conductive fiber fallout, in accordance with the present disclosure, the slitting can be performed in one step. For instance, one example of a slitting process in accordance with the present disclosure is shown in **FIG. 9****.** The system illustrated in **FIG. 9** is adapted to enclose and contain any free conductive fibers that may fall out from the material.

As shown in **FIG. 9**, a parent roll **84** comprised of the conductive nonwoven material 85 made according to the present disclosure is unwound into the process. In one embodiment, the parent roll **84** is center driven unwound so that no equipment is contacting the surface of the web. Surface driven unwind devices, on the other hand, can slip and cause surface roughness and can cause inconsistent feed rates which may result in wet breaks.

From the parent roll **84,** the nonwoven material **85** is first fed to a slitting device **86.** The slitting device, for instance, may comprise a rotary slitter that slits the entire width of the nonwoven material simultaneously. The rotary slitter **86,** for instance, may include rotary blades that are spaced apart a desired amount for forming the resulting slits.

As shown in **FIG. 9****,** in one embodiment, after the material is cut, the resulting slits can be separated into a first group of slits **87** and a second group of slits **88.** In one embodiment, for instance, the slits are divided in an alternative fashion in order to increase the spacing between the individual slits contained in each group. Thus, one half of the slits can be fed overhead to form the first group of slits **87** while the other half of the slits can be fed below to form the second group of slits **88.**

The first group of slits **87** are then wound onto a first set of spools **89,** while the second group of slits **88** is wound on a second set of spools **90.** The first set of spools **89,** for instance, includes the spools **91.** The second set of spools **90,** on the other hand, includes the spools **92.** In the embodiment illustrated, each set of spools shows four individual spools. It should be understood, however, that more or less spools can be included in the system depending upon the number of slits that are produced.

As the group of slits **87** are fed downstream, each individual slit is then wound on a corresponding spool **91.** For example, a single slit **95** is shown being wound on the last spool **91.**

In order to be fed onto the spool, the slit is passed around a guide roll **96** and then fed to a tension control device **93.** The tension control device **93** maintains constant tension on the slit during the winding process. Due to the relatively high tensile strength of the material, for instance, small cross tensions on the web during converting and winding on the spools may cause the slits to break. Thus, in one embodiment, a tension control device can be associated with each slit for maintaining constant tension.

In one embodiment, for instance, the tension control device **93** may comprise a dancer roll that applies a force to the slit **95** for maintaining the slit under a constant and uniform tension.

As shown in **FIG. 9****,** the slit **95** is wound onto the spool **91.** Once wound on the spool, the slit can be unwound into a separate process for the production of a particular article or product. In one embodiment, the slit can be traverse wound onto the spool **91.** Traverse winding takes the slit **95** and applies it to the spool core by traversing the length of the core. Traverse winding builds even and uniform rolls for later unwinding.

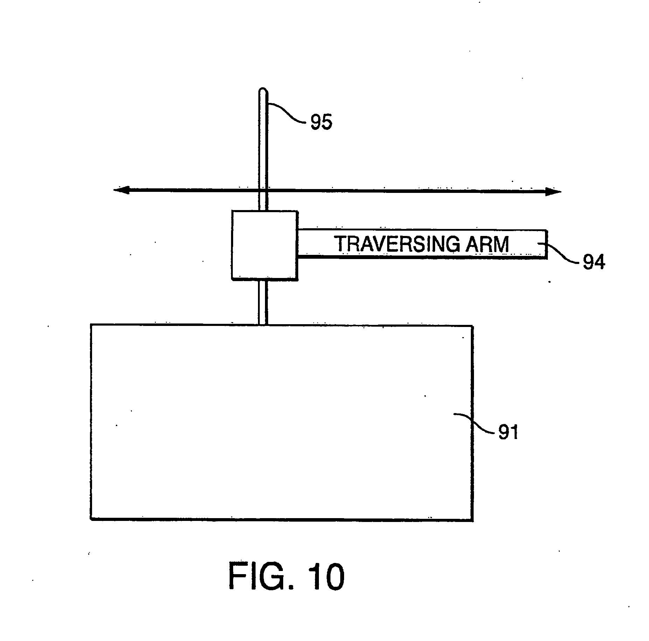

For example, referring to **FIG. 10****,** the slit **95** is shown being wound onto the spool **91.** As shown in greater detail, the system can include a traversing arm **94** that moves back and forth in relation to the spool **91** as the slit **95** is wound on the spool.

As described above, nonwoven base webs made in accordance with the present disclosure may be used in numerous applications. For instance, the base webs may be used for their ability to conduct electric currents.

In one particular application, for instance, the conductive nonwoven web may be incorporated into a wetness sensing device that is configured to indicate the presence of a body fluid within an absorbent article. The wetness sensing device, for instance, may comprise an open circuit made from the conductive nonwoven material. The open circuit can be connected to a signaling device which is configured to emit an audible, visual or sensory signal when a conductive fluid closes the open circuit.

The particular targeted conductive fluid or body fluid may vary depending upon the particular type of absorbent article and the desired application. For instance, in one embodiment, the absorbent article comprises a diaper, a training pant, or the like and the wetness sensing device is configured to indicate the presence of urine. Alternatively, the wetness signaling device may be configured to indicate the presence of a metabolite that would indicate the presence of a diaper rash. For adult incontinence products and feminine hygiene products, on the other hand, the wetness signaling device may be configured to indicate the presence of a yeast or of a particular constituent in urine, such as a polysaccharide.

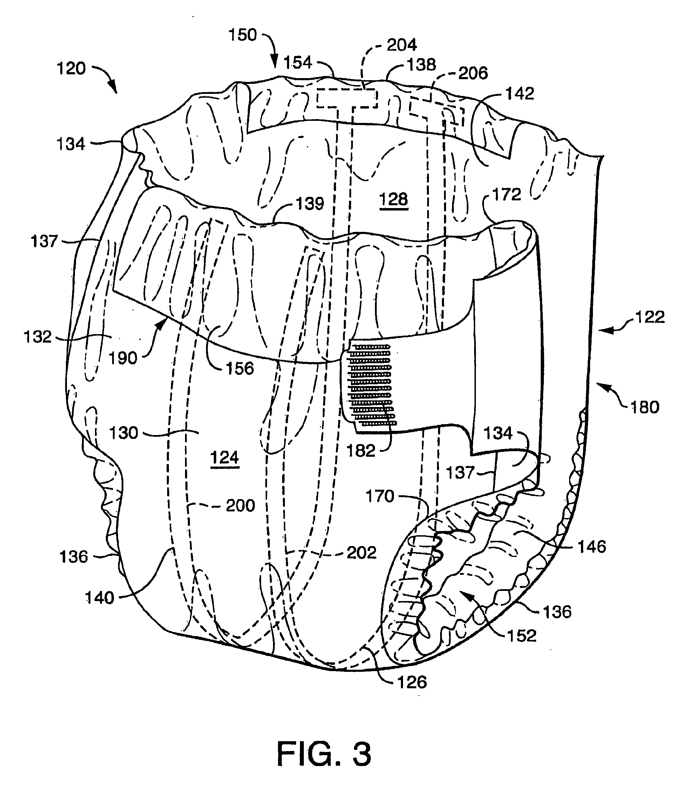

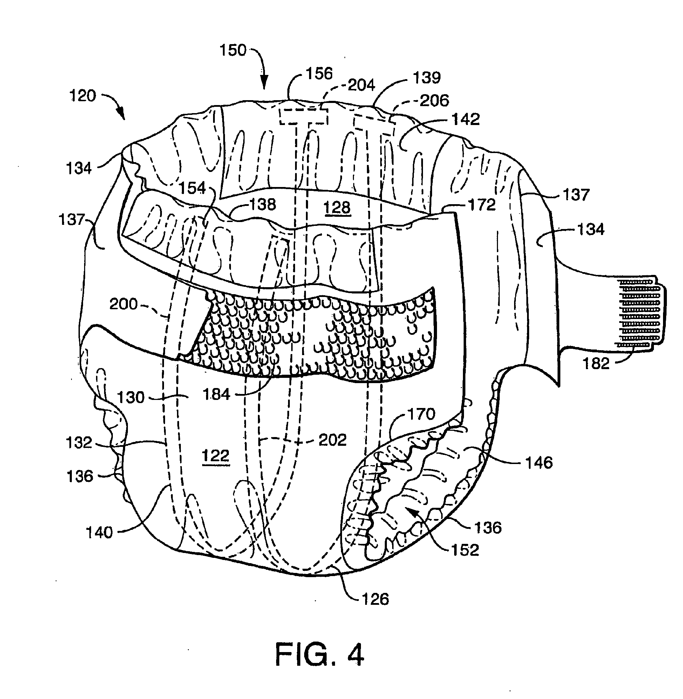

Referring to **Figs. 3** and **4****,** for exemplary purposes, an absorbent article **120** that may be made in accordance with the present invention is shown. The absorbent article **120** may or may not be disposable. It is understood that the present invention is suitable for use with various other absorbent articles intended for personal wear, including but not limited to diapers, training pants, swim pants, feminine hygiene products, incontinence products, medical garments, surgical pads and bandages, other personal care or health care garments, and the like without departing from the scope of the present invention.

By way of illustration only, various materials and methods for constructing absorbent articles such as the diaper **120** of the various aspects of the present invention are disclosed in PCT Patent Application WO 00/37009 published June 29, 2000 by A. Fletcher et al; U.S. Patent 4,940,464 issued July 10, 1990 to Van Gompel et al.; U.S. Patent 5,766,389 issued June 16, 1998 to Brandon et al., and U.S. Patent 6,645,190 issued November 11, 2003 to Olson et al..

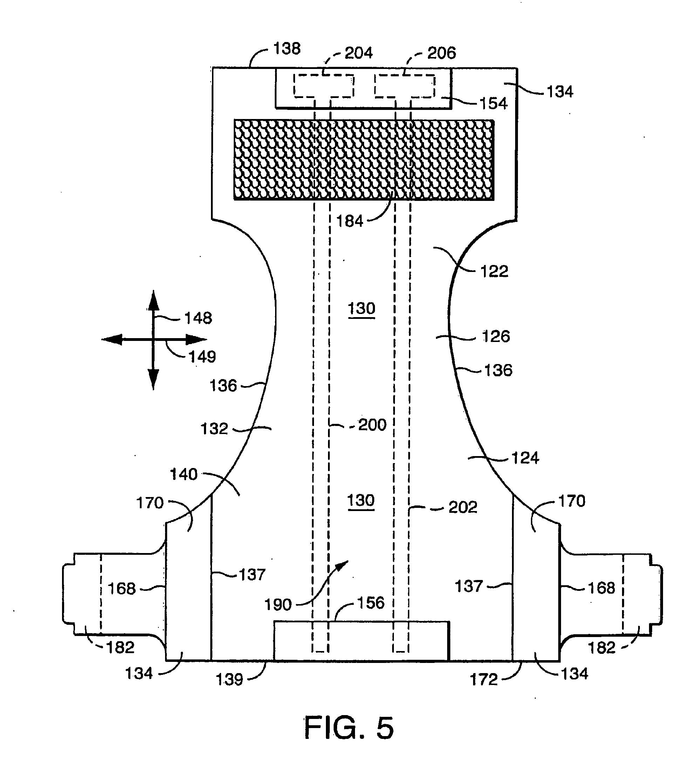

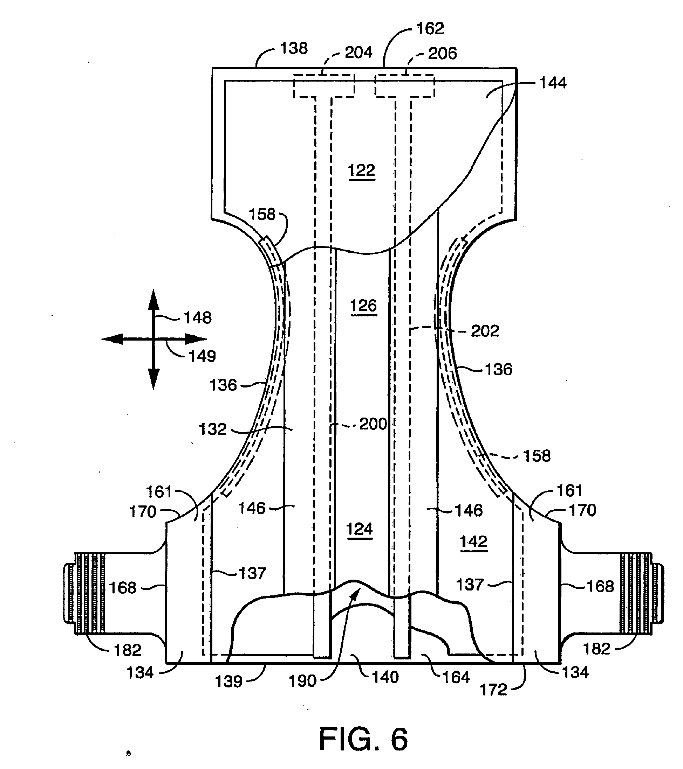

A diaper **120** is representatively illustrated in **Fig. 3** in a partially fastened condition. The diaper **120** shown in **Figs. 3** and **4** is also represented in **Figs. 5** and **6** in an opened and unfolded state. Specifically, **Fig. 5** is a plan view illustrating the exterior side of the diaper **120,** while **Fig. 6** illustrates the interior side of the diaper **120.** As shown in **Figs. 5** and **6****,** the diaper **120** defines a longitudinal direction **148** that extends from the front of the article when worn to the back of the article. Opposite to the longitudinal direction **148** is a lateral direction **149.**

The diaper **120** defines a pair of longitudinal end regions, otherwise referred to herein as a front region **122** and a back region **124,** and a center region, otherwise referred to herein as a crotch region **126,** extending longitudinally between and interconnecting the front and back regions **122, 124.** The diaper **120** also defines an inner surface **128** adapted in use (e.g., positioned relative to the other components of the article **120**) to be disposed toward the wearer, and an outer surface **130** opposite the inner surface. The front and back regions **122, 124** are those portions of the diaper **120,** which when worn, wholly or partially cover or encircle the waist or mid-lower torso of the wearer. The crotch region **126** generally is that portion of the diaper **120** which, when worn, is positioned between the legs of the wearer and covers the lower torso and crotch of the wearer. The absorbent article **120** has a pair of laterally opposite side edges **136** and a pair of longitudinally opposite waist edges, respectively designated front waist edge **138** and back waist edge **139.**

The illustrated diaper **120** includes a chassis **132** that, in this embodiment, encompasses the front region **122,** the back region **124,** and the crotch region **126.** Referring to **Figs. 3-6****,** the chassis **132** includes an outer cover **140** and a bodyside liner **142** (**Figs. 3** **and** **6**) that may be joined to the outer cover **140** in a superimposed relation therewith by adhesives, ultrasonic bonds, thermal bonds or other conventional techniques. Referring to **Fig. 6****,** the liner **142** may suitably be joined to the outer cover **140** along the perimeter of the chassis **132** to form a front waist seam **162** and a back waist seam **164.** As shown in **Fig. 6****,** the liner **142** may suitably be joined to the outer cover **140** to form a pair of side seams **161** in the front region **122** and the back region **124.** The liner **142** can be generally adapted, i.e., positioned relative to the other components of the article **120,** to be disposed toward the wearer's skin during wear of the absorbent article. The chassis **132** may further include an absorbent structure **144** particularly shown in **Fig. 6** disposed between the outer cover **140** and the bodyside liner **142** for absorbing liquid body exudates exuded by the wearer, and may further include a pair of containment flaps **146** secured to the bodyside liner **142** for inhibiting the lateral flow of body exudates.

The elasticized containment flaps **146** as shown in **Fig. 6** define a partially unattached edge which assumes an upright configuration in at least the crotch region **126** of the diaper **120** to form a seal against the wearer's body. The containment flaps **146** can extend longitudinally along the entire length of the chassis **132** or may extend only partially along the length of the chassis. Suitable constructions and arrangements for the containment flaps **146** are generally well known to those skilled in the art and are described in U.S. Patent 4,704,116 issued November 3,1987 to Enloe.

To further enhance containment and/or absorption of body exudates, the diaper **120** may also suitably include leg elastic members **158** (**Fig. 6**), as are known to those skilled in the art. The leg elastic members **158** can be operatively joined to the outer cover **140** and/or the bodyside liner **142** and positioned in the crotch region **126** of the absorbent article **120.**

The leg elastic members **158** can be formed of any suitable elastic material. As is well known to those skilled in the art, suitable elastic materials include sheets, strands or ribbons of natural rubber, synthetic rubber, or thermoplastic elastomeric polymers. The elastic materials can be stretched and adhered to a substrate, adhered to a gathered substrate, or adhered to a substrate and then elasticized or shrunk, for example with the application of heat, such that elastic retractive forces are imparted to the substrate. In one particular aspect, for example, the leg elastic members **158** may include a plurality of dry-spun coalesced multifilament spandex elastomeric threads sold under the trade name LYCRA and available from Invista, Wilmington, Delaware, U.S.A.

In some embodiments, the absorbent article **120** may further include a surge management layer (not shown) which may be optionally located adjacent the absorbent structure **144** and attached to various components in the article **120** such as the absorbent structure **144** or the bodyside liner **142** by methods known in the art, such as by using an adhesive. A surge management layer helps to decelerate and diffuse surges or gushes of liquid that may be rapidly introduced into the absorbent structure of the article. Desirably, the surge management layer can rapidly accept and temporarily hold the liquid prior to releasing the liquid into the storage or retention portions of the absorbent structure. Examples of suitable surge management layers are described in U.S. Pat. No. 5,486,166; and U.S. Pat. No. 5,490,846. Other suitable surge management materials are described in U.S. Pat. No. 5,820,973.

As shown in **Figs. 3-6****,** the absorbent article **120** further includes a pair of opposing elastic side panels **134** that are attached to the back region of the chassis **132.** As shown particularly in **Figs. 3** and **4****,** the side panels **134** may be stretched around the waist and/or hips of a wearer in order to secure the garment in place. As shown in **Figs. 5** and **6****,** the elastic side panels are attached to the chassis along a pair of opposing longitudinal edges **137.** The side panels **134** may be attached or bonded to the chassis **132** using any suitable bonding technique. For instance, the side panels **134** may be joined to the chassis by adhesives, ultrasonic bonds, thermal bonds, or other conventional techniques.

In an alternative embodiment, the elastic side panels may also be integrally formed with the chassis **132.** For instance, the side panels **134** may comprise an extension of the bodyside liner **142,** of the outer cover **140,** or of both the bodyside liner **142** and the outer cover **140.**

In the embodiments shown in the figures, the side panels **134** are connected to the back region of the absorbent article **120** and extend over the front region of the article when securing the article in place on a user. It should be understood, however, that the side panels **134** may alternatively be connected to the front region of the article **120** and extend over the back region when the article is donned.

With the absorbent article **120** in the fastened position as partially illustrated in **Figs. 3** and **4****,** the elastic side panels **134** may be connected by a fastening system **180** to define a 3-dimensional diaper configuration having a waist opening **150** and a pair of leg openings **152.** The waist opening **150** of the article **120** is defined by the waist edges **138** and **139** which encircle the waist of the wearer.

In the embodiments shown in the figures, the side panels are releasably attachable to the front region **122** of the article **120** by the fastening system. It should be understood, however, that in other embodiments the side panels may be permanently joined to the chassis **132** at each end. The side panels may be permanently bonded together, for instance, when forming a training pant or absorbent swimwear.

The elastic side panels **134** each have a longitudinal outer edge **168,** a leg end edge **170** disposed toward the longitudinal center of the diaper **120,** and waist end edges **172** disposed toward a longitudinal end of the absorbent article. The leg end edges **170** of the absorbent article **120** may be suitably curved and/or angled relative to the lateral direction **149** to provide a better fit around the wearer's legs. However, it is understood that only one of the leg end edges **170** may be curved or angled, such as the leg end edge of the back region **124,** or alternatively, neither of the leg end edges may be curved or angled, without departing from the scope of the present invention. As shown in **Fig. 6****,** the outer edges **168** are generally parallel to the longitudinal direction **148** while the waist end edges **172** are generally parallel to the transverse axis **149.** It should be understood, however, that in other embodiments the outer edges **168** and/or the waist edges **172** may be slanted or curved as desired. Ultimately, the side panels **134** are generally aligned with a waist region **190** of the chassis.

The fastening system **180** may include laterally opposite first fastening components **182** adapted for refastenable engagement to corresponding second fastening components **184.** In the embodiment shown in the figures, the first fastening component **182** is located on the elastic side panels **134,** while the second fastening component **184** is located on the front region **122** of the chassis **132.** In one aspect, a front or outer surface of each of the fastening components **182, 184** includes a plurality of engaging elements. The engaging elements of the first fastening components **182** are adapted to repeatedly engage and disengage corresponding engaging elements of the second fastening components **184** to releasably secure the article **120** in its three-dimensional configuration.

The fastening components **182, 184** may be any refastenable fasteners suitable for absorbent articles, such as adhesive fasteners, cohesive fasteners, mechanical fasteners, or the like. In particular aspects the fastening components include mechanical fastening elements for improved performance. Suitable mechanical fastening elements can be provided by interlocking geometric shaped materials, such as hooks, loops, bulbs, mushrooms, arrowheads, balls on stems, male and female mating components, buckles, snaps, or the like.

In the illustrated aspect, the first fastening components **182** include hook fasteners and the second fastening components **184** include complementary loop fasteners. Alternatively, the first fastening components **182** may include loop fasteners and the second fastening components **184** may be complementary hook fasteners. In another aspect, the fastening components **182, 184** can be interlocking similar surface fasteners, or adhesive and cohesive fastening elements such as an adhesive fastener and an adhesive-receptive landing zone or material; or the like. One skilled in the art will recognize that the shape, density and polymer composition of the hooks and loops may be selected to obtain the desired level of engagement between the fastening components **182, 184.** Suitable fastening systems are also disclosed in the previously acknowledged PCT Patent Application WO 00/37009 published June 29, 2000 by A. Fletcher et al. and the previously acknowledged U.S. Patent 6,645,190 issued November 11, 2003 to Olson et al.

In the embodiment shown in the figures, the fastening components **182** are attached to the side panels **134** along the edges **168.** In this embodiment, the fastening components **182** are not elastic or extendable. In other embodiments, however, the fastening components may be integral with the side panels **134.** For example, the fastening components may be directly attached to the side panels **134** on a surface thereof.

In addition to possibly having elastic side panels, the absorbent article **120** may include various waist elastic members for providing elasticity around the waist opening. For example, as shown in the figures, the absorbent article **120** can include a front waist elastic member **154** and/or a back waist elastic member **156.**

As described above, the present disclosure is particularly directed to incorporating a body fluid indicating system, such as a wetness sensing device into the absorbent article **120.** In this regard, as shown in **Figs. 3-6****,** the absorbent article **120** includes a first conductive element **200** spaced from a second conductive element **202.** In this embodiment, the conductive elements extend from the front region **122** of the absorbent article to the back region **124** without intersecting. In accordance with the present disclosure, the conductive elements **200** and **202** can be made from a conductive nonwoven material as described above. In the embodiment illustrated in **Fig. 4****,** the conductive elements **200** and **202** comprise separate and distinct strips or sheets. The strips, for instance, may comprise the slits shown in **FIG. 9** that may have a width, for example, of from about 3 mm to about 12 mm.

The first conductive element **200** does not intersect the second conductive element **202** in order to form an open circuit that may be closed, for instance, when a conductive fluid is positioned in between the conductive elements. In other embodiments, however, the first conductive element **200** and the second conductive element **202** may be connected to a sensor within the chassis. The sensor may be used to sense changes in temperature or may be used to sense the presence of a particular substance, such as a metabolite.

In the embodiment shown in **Fig. 3****,** the conductive elements **200 and 202** extend the entire length of the absorbent article **120.** It should be understood, however, that in other embodiments the conductive elements may extend only to the crotch region **126** or may extend to any particular place in the absorbent article where a body fluid is intended to be sensed.

The conductive elements **200** and **202** may be incorporated into the chassis **132** at any suitable location as long as the conductive elements are positioned so as to contact a body fluid that is absorbed by the absorbent article **120.** In this regard, the conductive elements **200** and **202** generally lie inside the outer cover **140.** In fact, in one embodiment, the conductive elements **200** and **202** may be attached or laminated to the inside surface of the outer cover **140** that faces the absorbent structure **144.** Alternatively, however, the conductive elements **200** and **202** may be positioned on the absorbent structure **144** or positioned on the liner **142.**

In order for the conductive elements **200** and **202** to be easily connected to a signaling device, the first conductive element **200** can include a first conductive pad member **204,** while the second conductive element **202** can include a second conductive pad member **206.** The pad members **204** and **206** are provided for making a reliable connection between the open circuit formed by the conductive elements and a signaling device that is intended to be installed on the chassis by the consumer.

The position of the conductive pad members **204** and **206** on the absorbent article **120 can** vary depending upon where it is desired to mount the signaling device. For instance, in **Figs. 3****,** **5** and **6****,** the conductive pad members **204** and **206** are positioned in the front region **122** along the waist opening of the article. In **Fig. 4****,** on the other hand, the conductive pad members **204** and **206** are positioned in the back region **24** along the waist opening of the article. It should be appreciated, however, that in other embodiments, the absorbent article **20** may include conductive pad members being positioned at each end of each conductive element **200** and **202.** In this manner, a user can determine whether or not to install the signaling device on the front or the back of the article. In still other embodiments, it should be understood that the pad members may be located along the side of the article or towards the crotch region of the article.

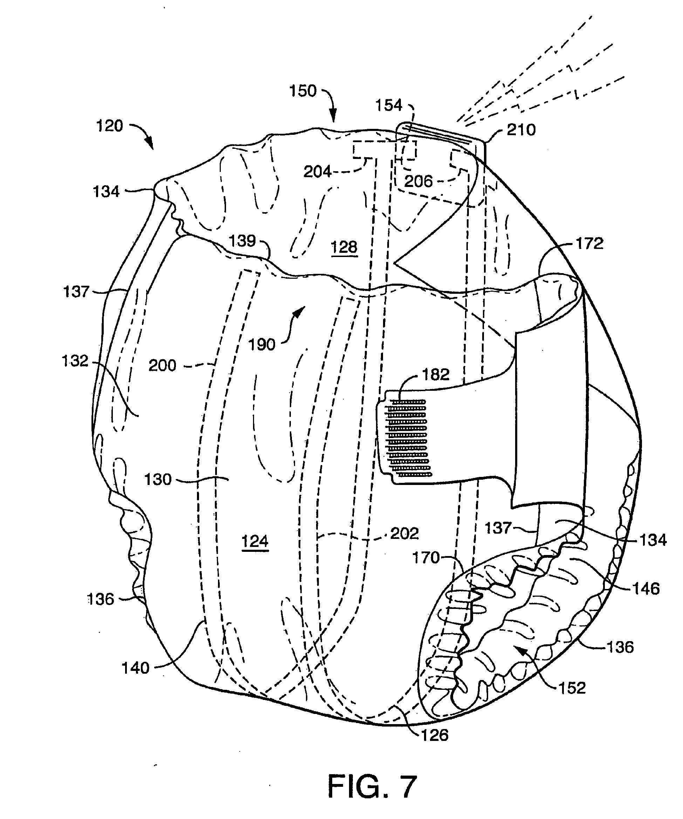

Referring to **Fig. 7****,** for exemplary purposes, a signaling device **210** is shown attached to the conductive pad members **204** and **206.** The signaling device **210** includes a pair of opposing terminals that are electrically connected to the corresponding conductive pad members. When a body fluid is present in the absorbent article **120,** the open circuit formed by the conductive elements **200** and **202** is closed which, in turn, activates the signaling device **210.**

The signaling device **210** can emit any suitable signal in order to indicate to the user that the circuit has been closed.

### EXAMPLE

For exemplary purposes only, the following demonstrates the conductivity of base webs made in accordance with the present disclosure.

A conductive nonwoven web was made according to the present disclosure containing conductive carbon fibers. The conductive nonwoven web was made on a Fourdrinier 36" paper machine, which is located at the publicly accessible HERTY Advanced Materials Development Center located in Savannah, GA.

A single layered web was produced containing a homogeneous blend of northern bleached softwood kraft fibers (LL19 from Terrace Bay Pulp Inc.), southern softwood kraft fibers (eucalyptus from Aracruz Celulose) and carbon fibers. The carbon fiber used was TENAX 150 fibers obtained from Toho Tenax having a cut length of 3 mm. The fiber furnish used to produce the web contained 94% by weight wood pulp fibers and 6% by weight carbon fibers. The wood pulp fiber blend contained 75% by weight softwood and 25% by weight hardwood.

The softwood furnish was refined using a 16" Beloit DD refiner with Finebar tackle to 365 CSF. The hardwood furnish was refined using 12" Sprout Twin Flow refiner to 365 CSF. Kymene 6500 from Hercules (Wilmington, DE) was added to the furnish at 10 kilograms per metric ton of dry wood pulp fibers. The consistency of the stock fed to the headbox was about 2.43 weight%.

The formed conductive nonwoven web was also coated on both sides with starch PG280 from Penford Products (Cedar Rapids, IA) and latex CP620NA (a carboxylated styrene-butadiene latex) from Dow Chemical (Midland, MI) as shown in Table below.

In producing the samples, the wet formed web was contacted with a first set of dryer cans. After the first set of dryer cans, the web was fed through a size press and then contacted with a second set of dryer cans.

Process conditions for the samples were:

| | | **Sample 1** | **Sample 2** | **Sample 3** |
|---|---|---|---|---|
| Machine Speed, FPM | | 200 | 200 | 200 |
| Primary Thick Stock Flow, GPM | | 25 | 25 | 50 |
| Primary Total Flow, GPM | | 200 | 200 | 200 |
| Holey Rolls, Direction | | F | F | F |
| Holey Rolls, RPM | | 1800 | 1800 | 1800 |
| Primary H.B. Level, in. | | 5 | 5 | 5 |
| Shake, % | | 90 | 90 | 90 |
| Vacuum, Inches of Water | | | | |
| Foil Box | #1 | 0 | 0 | 0 |
| | #2 | 8 | 9 | 9 |
| | #3 | 12 | 12 | 12 |
| | #4 | 22 | 20 | 20 |
| | #5 | 24 | 22 | 22 |
| Vacuum Flat Box No.1 In. of Hg. | | 0 | 0 | 0 |
| | No. 2 | 1 | 1 | 1 |
| | No. 3 | 0 | 0 | 0 |
| Couch Roll, In. of Hg. | | 9 | 6 | 6 |
| First Press, PLI | | 280 | 280 | 280 |
| Second Press, PLI | | 980 | 980 | 980 |
| First Dryer Section, Steam Pressure, PSI | | 8 | 8 | 8 |
| Size Press, PLI | | - | 36 | 36 |
| Pickup rate, lbs/Mton | | - | 140 | 140 |
| Second Dryer Section, Steam Pressure, PSI | | 11 | 21 | 21 |

The resulting web was then tested for resistance. The following results were obtained:

| | **Sample 1** | **Sample 2** | **Sample 3** |
|---|---|---|---|
| Coating at the size press | None | 6 weight % add-on of PG280 | 10 weight % add-on of 50:50 mixture of PG2800 and CP620NA |
| Air dry basis weight (gsm) | 40 | 42 | 42 |
| Resistance (Ohms/square) | 70 | 80 | 81 |
| Bulk (cc/g) | 2.1 | 2.2 | 2.2 |
| Machine direction tensile strength (grams/in) | 7892 | 10297 | 10248 |

The samples were tested for tensile strength using a tensile tester manufactured by MTS of Eden Prairie, MN, equipped with TESTWORKS 3 software. The tester was set up with the following test conditions:
Gauge length = 75mm
Crosshead speed = 300mm/min.
Specimen width = 1 inch (25.4mm)
Peak load at break was recorded as the tensile strength of the material.

## Claims

1. A process for producing a conductive paper web comprising:
depositing an aqueous suspension of fibers onto a porous forming surface to form a wet web, the aqueous suspension of fibers comprising softwood fibers mixed with carbon fibers, the carbon fibers having a length of from about 1 mm to about 6 mm and having a purity of at least about 85%, the carbon fibers being present in an amount of from about 5% to about 15% based on the total weight of the fibers present;
flattening the web;
drying the web;
slitting the web into a plurality of slits, each slit being wound on a separate spool, the slits being wound traversely on the spools.

2. A process as defined in claim 1, wherein the slits have a width of from about 3 mm to about 10 mm.

3. A process as defined in claim 1 or 2, wherein the softwood fibers have a Canadian Standard Freeness of greater than about 350 mL and/or wherein the carbon fibers have a purity of at least about 85%, preferably at least about 88%.

4. A process as defined in claim 1, 2 or 3, wherein the carbon fibers are coated with a water soluble size that is removed from the carbon fibers as the web is formed.

5. A process as defined in any of claims 1-4, wherein the slits have a final bulk of less than 1 g/cc and/or wherein the web is flattened by calendaring at a pressure of at least about 950 PLI.

6. A process as defined in any of claims 1-5, wherein an aqueous suspension of pulp fibers is formed first and then carbon fibers are injected into the aqueous suspension prior to depositing the fibers onto the forming surface.

7. A process as defined in any of claims 1-6, further comprising the step of incorporating a wet strength agent into the web.

8. A process as defined in claim 7, wherein the wet strength agent comprises a polyaminoamide-epichlorohydrin resin.

9. A process as defined in any of claims 1-8, wherein the dried web is wound into a roll, the roll then being center driven unwound as the web is slit.

10. A process as defined in claim 9, wherein the plurality of slits are divided in an alternative fashion to from a first group of slits and a second group of slits, the first group of slits being fed to a first set of corresponding spools, the second group of slits being fed to a second group of corresponding spools.

11. A process as defined in any of claims 1 to 10, wherein each spool is in association with a corresponding web tension device for controlling the tension of the slits as they are wound on the corresponding spools.

12. A process as defined in claim 11, wherein the tension device contains a dancer roll that is in communication with the slits as they are being wound on the spools, the process further including a traversing arm that directs the slits onto the spools for traversly winding the slits on the spools.

13. A process as defined in any preceding claim, wherein the paper web contains pulp fibers in an amount of at least about 50% by weight, the paper web having a length direction and a width direction, the paper web having a length direction tensile strength of at least about 5900 gf, the paper web having a basis weight of less than about 40 gsm and being uncreped and the paper web having a bulk of less than about 1 cc/g.

14. A process as defined in any preceding claim, wherein the paper web has a basis weight of at least about 15 gsm, and/or wherein the paper web contains softwood fibres in an amount of at least about 85% by weight.

15. A process as defined in any preceding claim, wherein the paper web is dyed such as a shade of blue or a shade of purple.

## Patentansprüche

1. Verfahren zum Herstellen einer leitenden Papierbahn, umfassend:
Ablagern einer wässrigen Suspension von Fasern auf eine poröse Formungsfläche, um eine nasse Bahn zu formen, wobei die wässrige Suspension von Fasern Weichholzfasern gemischt mit Kohlefasern umfasst, wobei die Kohlefasern eine Länge von ungefähr 1 mm bis ungefähr 6 mm haben und eine Reinheit von zumindest ungefähr 85% haben, die Kohlefasern in einer Menge von ungefähr 5% bis ungefähr 15%, bezogen auf das Gesamtgewicht der vorliegenden Fasern, vorhanden sind;
Abflachen der Bahn;
Trocknen der Bahn;
Schlitzen der Bahn in mehrere Streifen, wobei jeder Streifen auf eine eigene Spule gewickelt ist, wobei die Streifen kreuzweise auf die Spulen gewickelt sind.

2. Verfahren nach Anspruch 1, wobei die Streifen eine Breite von ungefähr 3 mm bis ungefähr 10 mm haben.

3. Verfahren nach Anspruch 1 oder 2, wobei die Weichholzfasern eine Kanadische Standardfreiheit von mehr als ungefähr 350 mL haben und/oder wobei die Kohlefasern eine Reinheit von zumindest ungefähr 85% haben, vorzugsweise zumindest ungefähr 88%.

4. Verfahren nach Anspruch 1, 2 oder 3, wobei die Kohlefasern mit einer wasserlöslichen Schlichte ummantelt sind, welche von den Kohlefasern entfernt wird, sobald die Bahn geformt wird.

5. Verfahren nach einem der Ansprüche 1-4, wobei die Streifen eine endgültige Dichte von weniger als 1 g/cm³ haben und/oder wobei die Bahn durch Kalandern bei einem Druck von zumindest ungefähr 950 PLI abgeflacht wird.

6. Verfahren nach einem der Ansprüche 1-5, wobei eine wässrige Suspension von Papierbreifasern zuerst geformt wird und dann Kohlefasern in die wässrige Suspension, vor Ablagern der Fasern auf die Formungsfläche, eingespritzt werden.

7. Verfahren nach einem der Ansprüche 1-6, des Weiteren umfassend den Schritt eines Einarbeitens eines Nassfestigkeitsmittels in die Bahn.

8. Verfahren nach Anspruch 7, wobei das Nassfestigkeitsmittel ein Polyaminoamid-Epichlorhydrin-Harz umfasst.

9. Verfahren nach einem der Ansprüche 1-8, wobei die getrocknete Bahn zu einer Rolle gewickelt wird, wobei die Rolle dann mittig angetrieben abgewickelt wird, sobald die Bahn geschlitzt wird.

10. Verfahren nach Anspruch 9, wobei die mehreren Streifen abwechselnd geteilt sind, um eine erste Gruppe von Streifen und eine zweite Gruppe von Streifen zu bilden, wobei die erste Gruppe von Streifen einem ersten Satz entsprechender Spulen zugeleitet wird, die zweite Gruppe von Streifen einer zweiten Gruppe entsprechender Spulen zugeführt wird.

11. Verfahren nach einem der Ansprüche 1 bis 10, wobei jede Spule in Verbindung mit einer entsprechenden Bahnspannungsvorrichtung zum Steuern der Spannung der Streifen steht, während sie auf die entsprechenden Spulen gewickelt werden.

12. Verfahren nach Anspruch 11, wobei die Spannungsvorrichtung eine Tänzerrolle enthält, welche mit den Streifen in Kontakt steht, während diese auf die Spulen gewickelt werden, wobei das Verfahren des Weiteren einen Verfahrarm beinhaltet, welcher die Streifen auf den Spulen lenkt, um die Streifen kreuzweise auf die Spulen zu wickeln.

13. Verfahren nach einem der vorangehenden Ansprüche, wobei die Papierbahn Papierbreifasern in einer Menge von zumindest ungefähr 50 Gewichtsprozent enthält, wobei die Papierbahn eine Längsrichtung und eine Breitenrichtung hat, die Papierbahn eine Zugfestigkeit in Längsrichtung von zumindest ungefähr 5900 gf hat, die Papierbahn ein Flächengewicht von weniger als ungefähr 40 g/m² hat und nicht gekreppt ist und die Papierbahn eine Dichte von weniger als ungefähr 1 cm³/g hat.

14. Verfahren nach einem der vorangehenden Ansprüche, wobei die Papierbahn ein Flächengewicht von zumindest 15 g/m² hat und/oder wobei die Papierbahn Weichholzfasern in einer Menge von zumindest ungefähr 85 Gewichtsprozent enthält.

15. Verfahren nach einem der vorangehenden Ansprüche, wobei die Papierbahn gefärbt ist, wie beispielsweise in einem Blauton oder einem Lilaton.

## Revendications

1. Processus pour produire une bande de papier conducteur comprenant :
le dépôt d'une suspension aqueuse de fibres sur une surface de formation poreuse pour former une bande humide, la suspension aqueuse de fibres comprenant des fibres de bois tendre mélangées avec des fibres de carbone, les fibres de carbone ayant une longueur d'environ 1 mm à environ 6 mm et ayant une pureté d'au moins environ 85 %, les fibres de carbone étant présentes en une quantité d'environ 5 % à environ 15 % sur la base du poids total des fibres présentes ;
l'aplanissement de la bande ;
le séchage de la bande ;
la refente de la bande en une pluralité de bandelettes, chaque bandelette étant enroulée sur une bobine distincte, les bandelettes étant enroulées de manière transversale sur les bobines.

2. Processus selon la revendication 1, dans lequel les bandelettes ont une largeur d'environ 3 mm à environ 10 mm.

3. Processus selon la revendication 1 ou 2, dans lequel les fibres de bois tendre ont un indice d'égouttage Canadien Standard supérieur à environ 350 mL et/ou dans lequel les fibres de carbone ont une pureté d'au moins environ 85 %, de préférence au moins environ 88 %.

4. Processus selon la revendication 1, 2 ou 3, dans lequel les fibres de carbone sont revêtues d'un apprêt soluble dans l'eau qui est enlevé des fibres de carbone lorsque la bande est formée.

5. Processus selon l'une quelconque des revendications 1 à 4, dans lequel les bandelettes ont un volume final de moins de 1 g/cm³ et/ou dans lequel la bande est aplanie par calandrage à une pression d'au moins environ 950 PLI.

6. Processus selon l'une quelconque des revendications 1 à 5, dans lequel une suspension aqueuse de fibres de pulpe est formée d'abord et ensuite des fibres de carbone sont injectées dans la suspension aqueuse avant de déposer les fibres sur la surface de formation.

7. Processus selon l'une quelconque des revendications 1 à 6, comprenant en outre l'étape d'incorporation d'un agent pour la résistance du papier à l'état humide dans la bande.

8. Processus selon la revendication 7, dans lequel l'agent pour la résistance du papier à l'état humide comprend une résine de polyaminoamide-épichlorhydrine.

9. Processus selon l'une quelconque des revendications 1 à 8, dans lequel la bande séchée est enroulée en un rouleau, le rouleau étant alors déroulé entraîné par le centre lorsque la bande est refendue.

10. Processus selon la revendication 9, dans lequel la pluralité de bandelettes est divisée d'une façon alternée pour former un premier groupe de bandelettes et un second groupe de bandelettes, le premier groupe de bandelettes étant amené à un premier ensemble de bobines correspondantes, le second groupe de bandelettes étant amené à un second groupe de bobines correspondantes.

11. Processus selon l'une quelconque des revendications 1 à 10, dans lequel chaque bobine est en association avec un dispositif de tension de bande correspondant pour maîtriser la tension des bandelettes lorsqu'elles sont enroulées sur les bobines correspondantes.

12. Processus selon la revendication 11, dans lequel le dispositif de tension contient un rouleau danseur qui est en communication avec les bandelettes lorsqu'elles sont enroulées sur les bobines, le processus incluant en outre un bras traversant qui dirige les bandelettes sur les bobines pour enrouler de manière transversale les bandelettes sur les bobines.

13. Processus selon l'une quelconque des revendications précédentes, dans lequel la bande de papier contient des fibres de pulpe en une quantité d'au moins environ 50 % en poids, la bande de papier ayant une direction en longueur et une direction en largeur, la bande de papier ayant une résistance à la traction dans le sens de la longueur d'au moins environ 5 900 gf, la bande de papier ayant un grammage inférieur à environ 40 gsm et étant non crêpé et la bande de papier ayant un volume de moins d'environ 1 cm³/g.

14. Processus selon l'une quelconque des revendications précédentes, dans lequel la bande de papier a un grammage d'au moins environ 15 gsm, et/ou dans lequel la bande de papier contient des fibres de bois tendre en une quantité d'au moins environ 85 % en poids.

15. Processus selon l'une quelconque des revendications précédentes, dans lequel la bande de papier est teintée comme une nuance de bleu ou une nuance de pourpre.
